# EUROPÄISCHE PATENTSCHRIFT

(11) **EP 3 109 311 B1**
(45) Veröffentlichungstag und Bekanntmachung des Hinweises auf die Patenterteilung: **20.12.2017**
(21) Anmeldenummer: 15020102.8
(22) Anmeldetag: 24.06.2015
(51) Int. Cl.: C12M 1/00, C12M 1/107, C12P 39/00, C12P 5/02, C12P 7/42

(54) **IN MEHREREN MODI BETRIEBENE SOLARGASANLAGE**
SOLAR GAS INSTALLATION WHICH CAN BE OPERATED IN MULTIPLE MODES
INSTALLATION GAS/SOLAIRE EXPLOITÉE DANS DIFFÉRENTS MODES

(43) Veröffentlichungstag der Anmeldung: 28.12.2016
(73) Patentinhaber: Solaga UG, 12489 Berlin (DE)
(72) Erfinder: Feustel, David, 13057 Berlin (DE); Herzog, Benjamin, 10439 Berlin (DE); Bauerfeind, Johann, 10405 Berlin (DE); Boldt, Yannick, 13353 Berlin (DE)
(74) Vertreter: Herzog, Benjamin

(56) Entgegenhaltungen:
- DE-A1-102007 031 688
- DE-A1-102010 040 440
- US-A1- 2014 045 234
- GÜNTHER A. ET AL.: "Methane production from glycolate excreting algae as a new concept in the production of biofuels", BIORES. TECH., 2012, Seiten 454-457, XP002751582,

## Beschreibung

Die Erfindung betrifft ein Verfahren zur Abtrennung von organischem Material aus dem aeroben Abschnitt eines kombinierten Fermenters und dessen Umsetzung in Biomethan im anaeroben Abschnitt sowie eine Vorrichtung zur Durchführung des Verfahrens.

Die Produktion von Biogas aus Pflanzen ist eine vielversprechende Technologie zum Erreichen der Energiewende. Biogas stellt aufgrund seiner einfachen Speicherbarkeit und seines hohen Energiegehalts mit einen der effektivsten Energieträger dar. Seit 2012 stagniert jedoch aufgrund rechtlicher und räumlicher Einschränkungen nahezu der Bau neuer Biogasanlagen in Vorreiterländern wie Deutschland (Fachverband Biogas-Prognose vom Juni 2014, wonach die Zahl der installierten Anlagen um nur 61 auf 8005 2015 steigen wird). Problematisch ist neben hohem Ackerflächenverbrauch, der zunehmend die Kosten für den Anbau von Nahrungsmitteln erhöht, dem aufwendigem Wachstum sowie der Ernte der Pflanzen und der Aufreinigung des Biogases, die energieaufwendige zentrale Versorgung der Bevölkerung mit dem Energieträger. Im Vergleich zu Landpflanzen haben phototrophe Mikroorganismen (PTM), zu denen u.a. Cyanobakterien, Seegras und Tang gehören, höhere Wachstumsraten und eine flächen bezogen höhere Produktivität (Dismukes et al. 2008; Bericht des FHI 2011). Die Kultivierung von PTM steht in keinem Konflikt mit der Produktion von Nahrungsmitteln, da eine PTM-Kultivierung nicht an die Nutzung von Ackerland gebunden ist. Vielmehr sind PTM äußerst genügsam und können an Orten kultiviert werden, die für Ackerbau und Industrie nicht nutzbar sind, wie an versiegelten Flächen, Küstenregionen, auf ertragslosen Böden wie z.B. in der Wüste oder an Salzwasserquellen, die keine entsprechende Wasserqualität besitzen. PTM benötigen im weitesten Sinne nur Kohlenstoffdioxid, Sonne und Spurenelemente zum Leben und Wachsen. Die dadurch geschaffene Unabhängigkeit ermöglicht nicht zuletzt eine dezentrale Versorgung der Bevölkerung mit rentabler Energie.

PTM stellen mit schätzungsweise mehr als 45.000 Arten ein biotechnologisches und wirtschaftliches Potential dar, das bei Weitem noch nicht ausgeschöpft ist. Da sie vergleichbar mit Pflanzen Kohlenstoffdioxid zum Aufbau von organischem Material nutzen, welches bei der Verbrennung wieder frei wird, ist ein energetisches Recycling von Kohlenstoffdioxid möglich ohne zur weiteren Erderwärmung beizutragen. Die reine Vergärung von PTM zu Biogas ist nicht sehr effektiv, da das entstehende Biogas mit Ammoniak und Schwefelwasserstoff verunreinigt ist und die Aufreinigung des Biogases sowie die Vorbehandlung der PTM viel Energie kostet. Demgegenüber wurde erkannt, dass nichtwachsende PTM, die vergleichbar mit mikroskopischen Fabriken organisches bzw. anorganisches Material aufbauen, zur Produktion von reinem Biogas geeigneter sind. Sie benötigen keinen Dünger und der Energieaufwand ist nach dem Wachstum in der Produktionsphase gering. Biogas wird durch PTM selbst nicht hergestellt. Sie können aber Intermediate herstellen, die von einem Partnerorganismus weiter zu Biogas verstoffwechselt werden, wobei beide Prozesse insgesamt effektiver sind als die Herstellung eines Energieträgers durch PTM alleine.

Aus der DE102007031688A1 ist ein Verfahren bekannt bei dem Algen Wasserstoff und Sauerstoff aus Kohlenstoffdioxid und Wasser produzieren, welches in einem weiteren Schritt in Methan durch methanogene Bakterien umgewandelt wird. CN103571876A hat diese Methode durch eine Regulierung der störenden Sauerstoffabgabe der Algen verbessert. Beide Verfahren haben den Nachteil, dass erst energieaufwendig Stärke aufgebaut wird, aus der im weiteren Verlauf Wasserstoff und Sauerstoff entsteht. Es hat sich herausgestellt, dass die Energiegewinnung durch Photovoltaik effizienter ist als die Energiegewinnung durch Photosynthese bezogen auf die Synthese von Glucose (Blankenship R.E. et al. 2011). Bei natürlicher Lichteinstrahlung beträgt die Effizienz tatsächlich nur 1 bis 2 % (Barber J. 2009). Hinzu kommt der Energieverlust bei der Umwandlung in Wasserstoff, der zu einer Effizienz von 0,005 % bei den vergleichbaren Cyanobakterien führt. Nur durch genetische Modifikation wurde eine Effizienz von ca. 1 % erreicht (Masukawa H. et al. 2012).

DE102010040440A1 betrifft einen Bioreaktor, in dem photosynthetisch aktive Mikroorganismen mit Kohlenstoffdioxid und Sauerstoff versorgt werden und dabei Glykolat ausscheiden, welches durch eine Membran abgetrennt wird und unter anaeroben Bedingungen als Substrat für methanogene Mikroorganismen dient, die es in Kohlenstoffdioxid und Methan umwandeln. Die Methode hat den Nachteil, dass es keine ausschließlich für Glykolat selektive Membran gibt und dass eine Strippingkammer nicht ausreicht, um den Sauerstoffgehalt ausreichend zu reduzieren. Des Weiteren ist nicht geklärt, wie das atmosphärische Kohlenstoffdioxid in das System gelangt. Für die Nutzung einer Gasmischeinrichtung sind nicht zuletzt separate Gasspeicher mit dem jeweiligen Reinstgas notwendig, deren Befüllung zumindest die Energiebilanz beeinträchtigt. Weitere ungelöste Probleme sind die Versorgung der Mikroorganismen mit Nährgas bzw. Sauerstoff und die Nährstoffversorgung im Dauerbetrieb bei zwei verschiedenen Medien. Nicht zuletzt wird nicht berücksichtigt, dass unangepasste methanogene Bakterien durch organische Säuren angegriffen werden. Gegenstand der erfindungsgemäßen Vorrichtung nach DE102009008601A1 ist eine Vorrichtung mit drei Kammern, bei der zwischen der ersten und der zweiten Kammer ein Gasaustausch über eine gasdurchlässige und flüssigkeitsundurchlässige Membran zur Behandlung einer biologischen Flüssigkeit und ein Flüssigkeitsaustausch zwischen erster und dritter Kammer durch eine Membran erfolgt.

Durch die Erfindung soll das Problem gelöst werden, dass bei der Produktion von organischem Material in einem Teilbereich eines Fermenters hohe Sauerstoffpartialdrücke herrschen, wobei in einem weiteren Teilbereich, der mit dem ersten in Verbindung steht, diese hohen Sauerstoffpartialdrücke zu vermeiden sind. Weiterhin soll das Problem gelöst werden, dass Kohlenstoff aus der Luft in Energieträger im Rahmen eines Fermentationsprozesses ohne großen Energieaufwand integriert werden soll.

Erfindungsgemäß wird die Aufgabe durch das in Anspruch 1 beschriebene Verfahren gelöst, wobei die in Anspruch 11 beschriebene Vorrichtung zur Durchführung des Verfahrens geeignet ist.

Die Erfindung basiert auf der nachfolgend dargestellten wissenschaftlichen Grundlage.

PTM produzieren und sekretieren bei bestimmten physiologischen Bedingungen organisches Material, d.h. Kohlenstoffverbindungen wie zum Beispiel organische Carbonsäuren. In der Glycogensynthese behinderte Synechocystis sp. PCC 6803 stellen beispielsweise bei Stickstoffmangel ∟-Ketoglutarsäure und Brenztraubensäure her (Carrieri D. et al. 2015).

Bei im Vergleich zu Luft erhöhtem Sauerstoff-Kohlenstoffdioxid-Verhältnis erzeugen PTM im Calvinzyklus, durch den bei normalen Bedingungen Kohlenstoffdioxid fixiert und für den Aufbau von Glucose zur Energieversorgung genutzt wird, organisches Material, welches von besonderem biotechnologischem Interesse ist. Diesem Phänomen liegt die Tatsache zugrunde, dass Ribulose-1,5-bisphosphat-Carboxylase/Oxygenase (RuBisCO) alternativ zu Kohlenstoffdioxid auch Sauerstoff akzeptiert, wobei der KM-Wert für Sauerstoff mit 350 µMol/l höher als für Kohlenstoffdioxid mit 9 µMol/l ist. Vor langer Zeit war in der Erdatmosphäre der Sauerstoffgehalt noch so gering, dass dieser Prozess noch keine Rolle spielte. Bei der Reaktion mit Sauerstoff entsteht neben 3-Phosphoglycerinsäure (3-PGA) auch 2-Phosphoglycolsäure (2-PG), die nicht mehr im Calvin-Zyklus oder anderweitig im Organismus verwendet werden kann und daher durch andere biochemische Reaktionen recycelt oder sekretiert werden muss. Der zur Regeneration von Kohlenstoff für den Calvinzyklus genutzte Stoffwechselweg gilt als einer der verschwenderischsten Prozesse auf der Erde. Dieser Umstand ist andererseits für die dem erfindungsgemäßen Verfahren zugrundeliegende natürliche Biotechnologie nutzbar.

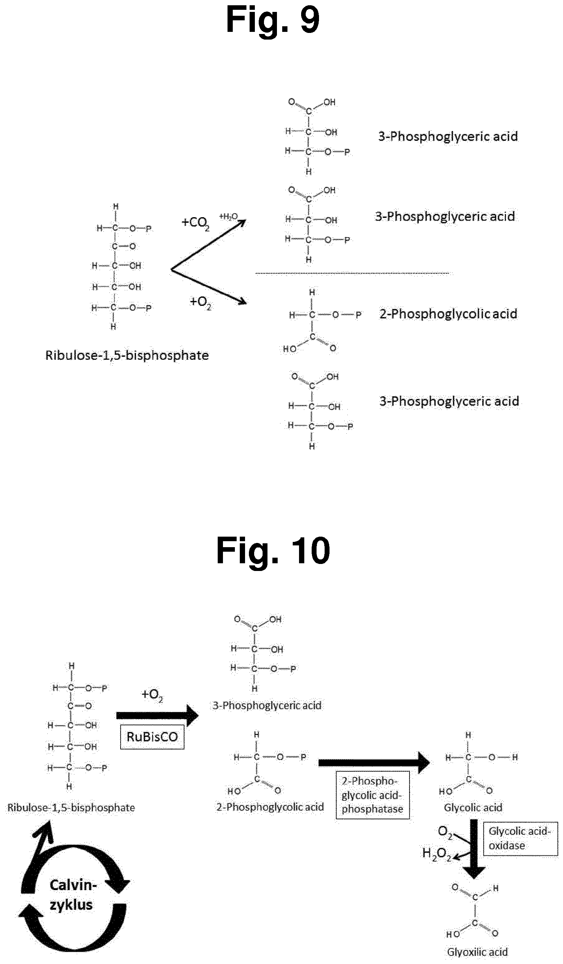

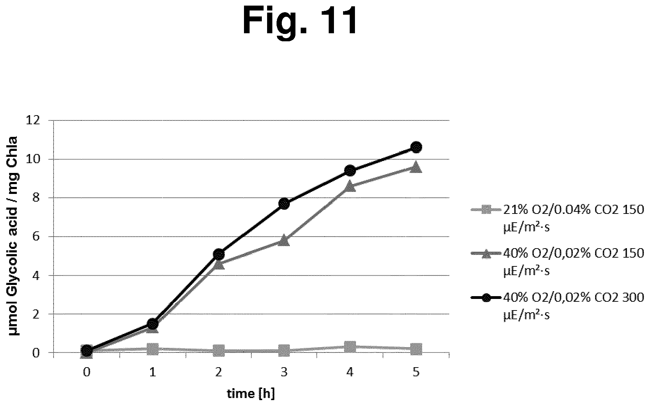

Der Umgang mit 2-PG, dem ersten stabilen Zwischenprodukt nach dem Calvinzyklus, ist von Organismus zu Organismus unterschiedlich. 2-PG wird durch Dephosphorylierung in Glycolsäure (Hydroxyessigsäure) umgewandelt, teils sekretiert (Figur 11) teils zu Glyoxylsäure verstoffwechselt (Figur 10). Diese wird wiederrum zum Teil ebenfalls sekretiert oder weiter verstoffwechselt, wobei teilweise Wasserstoffperoxid, Glycin, L-Serin, 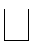 -Ketoglutarsäure mit Stickstoff-Donoren oder Glutaminsäure entstehen (Bauwe H. 2011). Im Unterschied zu Cyanobakterien finden die Stoffwechselwege in eukaryotischen PTM in verschiedenen Kompartimenten statt. Zum größten Teil wird aus 2-PG 3-PGA regeneriert und wieder in den Calvinzyklus geschleust, wobei Kohlenstoffdioxid frei bzw. "ausgeatmet" wird. Um weiterhin Glycolsäure zu produzieren, ist ein Auffüllen des Ribulose-1,5-bisphosphat-Pools notwendig, was nur durch zwischenzeitliche Kohlenstoffdioxidaufnahme geschehen kann.

Archaea, die Biomethan produzieren, sogenannte Methanbildner, nutzen organisches Material und bauen daraus Biomethan auf. Dabei unterscheidet man acetatspaltende (acetotrophe) und wasserstoffoxidierende (hydrogenotrophe) Methanbildner. Die acetotrophen Methanbildner spalten Methylgruppen aus organischem Material ab und reduzieren sie zu Biomethan. Dafür benutzen sie das Coenzym Methanophenazin. Unter ihnen finden sich unter anderem Methanosarcina. Es konnte schon 1973 gezeigt werde, dass anoxische Mikrorganismen nur mit Glycolsäure als einziger Kohlenstoffquelle wachsen können (Kurz W.G.W. et al. 1973; Edenborn H.M. et al. 1985). Friedrich M. et al. 1991 und Friedrich M. et al. 1996 entdeckten zwei acetotrophe Stämme, die auf Glycolsäure wachsen und in einer Mischkultur Methan herstellen, wobei gilt: 4 C2H4O3 (Glycolsäure) -> 3 CH4 + 5 Co2 + 2 H2O. Egli C. et al. 1989 zeigten, dass Methanbildner mit Monochlor- und Dichloracetat als Kohlenstoff- und Energiequelle leben können.

Hydrogenotrophe Methanbildner wie Methanococcus, Methanobacterium und Methanopyrus hingegen bilden Methan durch Reduktion von Kohlenstoffdioxid mit Wasserstoff zu Methan und Wasser oder durch Umwandlung von Ameisensäure. Sie verfügen nicht über das Enzym Methanophenazin. Der größte Teil der Methanbildner benötigt ein anoxisches, pH-neutrales oder schwach basisches Medium mit mindestens 50 % Wasser. Gewässerablagerungen, überwässerte Böden wie Moore und Reisfelder, Mist, Gülle und der Darm von Wiederkäuern stellen hervorragende Biotope für Methanbildner dar. Hemmstoffe für Methanbildner sind organische Säuren, Desinfektionsmittel und Sauerstoff.

Die Reaktionsgleichung für die Bildung von Biomethan im Laufe des erfindungsgemäßen Gesamtprozesses lautet: CO2 + 2H2O -> CH4 + 2O2.

Um Mikroorganismen an besondere Medienbestandteile, Ernährungsquellen und Stoffwechselendprodukte anderer Mikroorganismen zu gewöhnen, werden die Mikroorganismen stufenweise an die Bestandteile angepasst. Dies geschieht in einem Turbidostaten, der bei einem Abfall des Wachstums bzw. der Populationsdichte den Zufluss von Medium mit dem Bestandteil, an den sich gewöhnt werden soll, verringert und den Zufluss von für Wachstum besser geeignetem Medium erhöht. Bei diesem System wird nicht nur ausgenutzt, dass manche Mikroorganismen evolutionär bedingt über Stoffwechselwege verfügen, die in ihren jeweiligen bevorzugten Lebensräumen nicht gebrauchen. Es werden auch im Rahmen von Langzeitprojekten stabile Stämme durch Mutationen erzeugt. Durch diese Methode ist es möglich, Methanbildner an organisches Material insbesondere organische Säuren als einzige Kohlenstoff- bzw. Energiequelle ohne genetische Manipulation anzupassen.

Das erfindungsgemäße Verfahren ist effizienter als die Produktion von glucosebasierten Biokraftstoffen, bei der die Effizienz der Glucosesynthesehon nur 1-2 % beträgt (Linder H. 1998). Circa 36 % der eingestrahlten Sonnenenergie wird durch die Lichtreaktion der Photosynthese in ATP gespeichert, da vier Einstein Rotlicht mit jeweils 172 kJ 688 kJ entsprechen und für den Transport von 2 Mol Elektronen bzw. 218,9 kJ reichen, wodurch 1 Mol ATP mit 30,6 kJ entsteht (zusammen 249,5 kJ), welches für die Kohlenstofffixierung im Calvinzyklus gebraucht wird. Zusammen mit dem Verbrauch von NADPH beträgt die Effizienz des Calvinzyklus mit Aufbau und Sekretion ca. 25 % (Nabors M.W. et al. 2007). Die Methanbildung durch die Archaea ist mit ca. 70 % sehr effizient (Bernacchi S. et al. 2014). Der Gesamtwirkungsgrad der erfindungsgemäßen Bildung von Biomethan liegt im Ergebnis bei ca. 18 % der eingestrahlten Sonnenenergie. Damit ist der Wirkungsgrad höher als bei der Photovoltaik, welcher dort nur 16 % erreicht.

Im Folgenden wird die erfindungsgemäße Lösung geschildert. Das erfindungsgemäße Verfahren beruht auf einer Abfolge von Modi, die beginnend mit dem Produktionsmodus nachfolgend dargestellt sind.

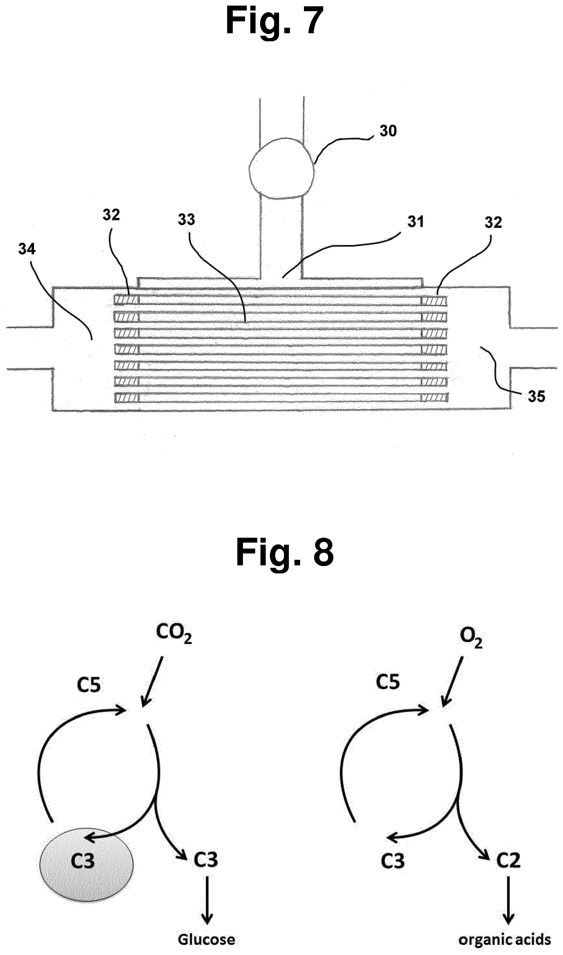

In dem erfindungsgemäßen Verfahren stellen PTM in einem ersten Abschnitt im Produktionsmodus organisches Material insbesondere Glycolsäure unter oxischen Bedingungen aus Kohlenstoffdioxid und Sauerstoff her und sekretieren es in ein Medium. Sie nutzen dabei Sonnenlicht und die im Medium vorhandenen Spurenelemente. Ihr Wachstum ist eingestellt. Sie benötigen keinen Dünger zum Wachsen oder andere für das Wachstum nötige Bestandteile im Medium. Das organische Material wird auf natürlichem Weg sekretiert. Es entsteht in den Zellen durch die Nutzung des Sauerstoffs anstelle von Kohlenstoffdioxid (rechte Seite in Figur 8).

Nach der Produktion des organischen Materials, wobei vorteilhaft eine Lichteinstrahlung von 100 bis 200 µE/m²·s von mehr als einer Stunde nötig ist, wird im Austauschmodus das Medium, welches das organische Material enthält, einem zweiten Abschnitt zugeleitet, in dem das organische Material von Methanbildnern unter anoxischen Bedingungen in Biomethan umgesetzt wird. Während des Übergangs zum zweiten Abschnitt wird das Medium von Sauerstoff entgast, damit der für die Methanbildner schädliche Sauerstoff nicht in den zweiten Abschnitt gelangt. Bis zu einem Restsauerstoffgehalt von 4 % sind die Methanbildner nicht gefährdet. Es wird beispielsweise bezogen auf Glykolsäure im Mittel 0,24 ml Biomethan pro mg Glykolsäure umgesetzt (Günther A. et al. 2012). In einem Gesamtfermenterkreislauf, d.h. in einem beide Abschnitte des kombinierten Fermenters betreffenden Kreislauf, wird beim Rücklauf zum ersten Abschnitt das Medium wieder mit Sauerstoff begast, damit die oxischen Bedingungen für die Produktion des organischen Materials im ersten Abschnitt wieder hergestellt werden. Das Medium, in dem sich die PTM hier beispielhaft Cyanobakterien wie Gloeothece 6909 und die Methanbildner wie Clostridien wie Synthrophobotulus glycolicus oder FIGlyM (Friedrich et al. 1996) sowie Methanococcus maripuladis befinden, hat vorzugsweise zumindest folgende Zusammensetzung:

| **Medienbestandteil** | **Menge in g/l** |
|---|---|
| KH₂PO₄ | 0,12 |
| K₂HPO₄ | 0,12 |
| NaNO₃ | 0,15 |
| KCl | 0,42 |
| NH₄Cl | 0,25 |
| CaCl₂ x 2 H₂O | 0,12 |
| Resazurin | 0,75·11⁻³ |
| Na₂S x 9 H₂O | 0,40 |
| NaHCO₃ | 3,75 |
| L-Cysteine-HCl x H₂O | 0,40 |
| EDTA | 0,005 |
| NaCl | 4,50 |
| MgCl₂ x 6 H₂O | 1 |
| Spurenelementlösung SL-10 (nach Wolfe) | 1 ml |
| Vitaminlösung (nach DSMZ-Medium 141) | 10 ml |

Das Medium ergibt sich aus den vom DSMZ, Leibniz Institut - Deutsche Sammlung von Mikroorganismen und Zellkulturen GmbH, empfohlenen Bestandteilen und dem für Cyanobakterien geeigneten Bold's Basal Medium (Stein J. 1973).

In einer bevorzugten Ausführungsform des erfindungsgemäßen Verfahrens wird, während der Kontakt zum zweiten Abschnitt unterbrochen ist, das Medium von Sauerstoff entgast und bei einem Rücklauf mit Luft aus der Umgebung begast, danach die Luft von den PTM zur Kohlenstoffassimilation genutzt (Regenerationsmodus) und anschließend das Medium von Luft entgast und bei einem Rücklauf mit Sauerstoff begast wird. Nach dem Verbrauch des organischen Materials im zweiten Abschnitt und dem Verbrauch von Kohlenstoffverbindungen, die als Ausgangsmaterial für die Produktion des organischen Materials in den PTM dienen und insbesondere im reduktiven Pentosephosphatweg vorhanden sind, vorteilhaft nach der doppelten bis vierfachen Zeit der Lichteinstrahlung im Produktionsmodus, ist vorteilhaft der Regenerationsmodus einzuleiten. Er dient der Auffüllung des C3-Pools im Calvinzyklus (Lorimer G.H. et al. 1981) und der eigenen Kohlenstoffversorgung der PTM (Figur 8). Im Produktionsmodus entsteht bei einem höheren Sauerstoffanteil neben 2-PG auch 3-PGA (Figur 9), welches wieder in den Calvinzyklus eingeht. Netto geht dem Calvinzyklus jedoch bei jeder Reaktion der RuBisCO Kohlenstoff verloren. Um diesen Verlust auszugleichen, ist es notwendig zwischenzeitlich den Sauerstoffgehalt zugunsten von Kohlenstoffdioxid zu verringern. Dazu eignet sich am besten die atmosphärische Luft, deren erderwärmendes Kohlenstoffdioxid verbraucht werden soll. Bei Anwesenheit des Kohlenstoffdioxids wird dieser ausschließlich in 3-PGA umgesetzt. Ein Teil davon wird für die Regenerierung des C3-Pools und der andere Teil zum Aufbau von körpereigenen Kohlenstoffverbindungen wie Glucose zur Versorgung des Organismus genutzt (Figur 8). Im Regenerationsmodus ist der Kontakt zum zweiten Abschnitt unterbrochen. Die PTM werden beleuchtet, da die Enzyme des Calvinzyklus nur bei Beleuchtung aktiv sind.

Beim Übergang vom Produktionsmodus mit einem hohen relativen Sauerstoffgehalt im Medium zum Regenerationsmodus mit gelöster Luft im Medium wird das Medium bevorzugt aus dem ersten Abschnitt abgeleitet und von Sauerstoff entgast. Anschließend wird das Medium vorzugsweise mit Luft begast und dem ersten Abschnitt wieder zugeleitet, wodurch ein Teilreaktorkreislauf etabliert wird. Der Austausch mit dem zweiten Abschnitt ist dabei bevorzugt unterbrochen.

Der Übergang vom Regenerationsmodus zum Produktionsmodus wird vorzugsweise im Teilreaktorkreislauf bei unterbrochenem Kontakt zum zweiten Abschnitt durch Entgasung des Mediums von Luft und Begasung mit Sauerstoff vorgenommen.

In einer bevorzugten Ausführungsform des erfindungsgemäßen Verfahrens ist das Verhältnis von Kohlenstoffdioxid zu Sauerstoff in dem im ersten Abschnitt befindlichen Medium im Produktionsmodus gering, bevorzugt 1:800 bis 1:3000. Dies entspricht dem Bereich, in dem die RuBisCO vermehrt Sauerstoff akzeptiert und die Vorläufer für organisches Material durch die PTM produziert werden ohne, dass der Sauerstoffgehalt toxisch wirkt. Im Gegensatz dazu ist das Verhältnis im Regenerationsmodus entsprechend der Luftzusammensetzung 1:500. Die Verringerung des Kohlenstoffdioxid-Sauerstoff-Verhältnis wird erreicht durch Entgasung der Luft und Begasung mit Sauerstoff im Übergang zum Produktionsmodus.

In einer bevorzugten Ausführungsform des erfindungsgemäßen Verfahrens wird im Austauschmodus das Medium beim Übergang vom ersten Abschnitt zum zweiten Abschnitt nach der Sauerstoffentgasung mit einem Ersatzgas aus einem Ersatzgasspeicher insbesondere mit Kohlenstoffdioxid oder Stickstoff begast. Dadurch kommen die Methanbildner nicht in Kontakt mit Sauerstoff, sondern mit einem für sie unschädlichen Gas. Das Kohlenstoffdioxid kann insbesondere aus dem Biogas zurückgewonnen werden und zunächst dem Ersatzgasspeicher zugeführt werden. Stickstoff ist ebenfalls als Ersatzgas aufgrund seines inerten Charakters geeignet und auch in der Luft ubiquitär vorhanden. Beim Rücklauf vor der Sauerstoffbegasung wird das Medium von dem Ersatzgas wieder entgast. Das Medium ist im entgasten Zustand aufnahmefähig und -bedürftig für den Sauerstoff. Somit können wieder hohe Sauerstoffpartialdrücke im ersten Abschnitt erreicht werden. Das zurückgewonnene Gas wird wieder im Ersatzgasspeicher gespeichert, so dass es nicht verloren geht und für die Begasung wiederverwendet werden kann.

In einer bevorzugten Ausführungsform des erfindungsgemäßen Verfahrens wird von dem im zweiten Abschnitt entstehenden Biogas durch einen Biogasfilter das Kohlenstoffdioxid abgetrennt und in dem Ersatzgasspeicher zur beschriebenen Ersatzbegasung gespeichert. Darüber hinaus kann bei übermäßiger Kohlenstoffdioxidanreicherung im Ersatzgasspeicher das Kohlenstoffdioxid im Regenerationsmodus anstelle der Luft benutzt werden. Die Auftrennung von Biogas in Biomethan und Kohlenstoffdioxid erfolgt bevorzugt durch einen Hohlfaserkontaktor. Dabei wird Kohlenstoffdioxid aus der gasförmigen Faser durch die Poren der Hohlfasern, die kleiner als 0,05 µm sind, abgetrennt, während das langsamer durchtretende Biomethan in der Hohlfaser verbleibt und weitergeleitet wird. Es kann dann in einem Biomethanspeicher gespeichert und ohne weiteres zur Energiegewinnung genutzt werden. Vorteilhaft ist insbesondere, dass die Konzentrationen an giftigen Stickstoffverbindungen wie Stickoxiden und Ammoniak sowie Schwefelverbindungen wie Schwefelwasserstoff vernachlässigbar gering vorzugsweise unter 0,1 % liegen.

In einer bevorzugten Ausführungsform des erfindungsgemäßen Verfahrens werden zur Be- und Entgasung Filter insbesondere Hohlfaserkontaktoren verwendet, wobei die Porengröße unter 0,1 µm bevorzugt unter 0,04 µm beträgt. Die vorzugsweise zu benutzenden Hohlfaserkontaktoren zeichnen sich durch eine hohe Effektivität und einen geringen Energieaufwand bei der Be- und Entgasung von Flüssigkeiten aus. Bei der Entgasung wird das Medium durch die hydrophoben Hohlfasern geleitet, die in hoher Zahl in dem Kontaktor am Anfang und am Ende gebündelt vorhanden sind (Figur 7). Durch die hohe Anzahl der Fasern wird eine sehr große Oberfläche erreicht. Aufgrund der geringen Porengröße und der Hydrophobizität treten nur die Gasmoleküle durch die Membran, was durch einen Unterdruck verstärkt wird. Bei der Begasung ist es umgekehrt, d.h. durch einen Überdruck werden die Gasmoleküle in die Flüssigkeit gedrückt, ohne dass Flüssigkeitsmoleküle auf die andere Seite gelangen können. Für Kohlenstoffdioxid und Sauerstoff werden aufgrund ihrer unterschiedlichen Eigenschaften bevorzugt unterschiedliche Kontaktoren mit folgenden Eigenschaften der Hohlfasern verwendet:

| **Membrankontaktor für** | **Kohlenstoffdioxid** | **Sauerstoff** |
|---|---|---|
| Aussendurchmesser (in µm) | 300 | 300 |
| Innendurchmesser (in µm) | 200 | 220 |
| Blaspunkt (in psi) | 240 | 240 |
| Porosität (in %) | 25 | 40 |
| Porengröße (in µm) | 0,03 | 0,04 |

Die bevorzugt zu benutzenden Hohlfaserkontaktoren für die Entgasung und die Begasung weisen einen Eingang und einen Ausgang für den Flüssigkeitsstrom auf. Dem angeschlossen ist jeweils ein Eingangsraum und ein Ausgangsraum. Dazwischen befinden sich die Hohlfasern, welche durch eine Trennwand beidseitig fixiert sind. Die Trennwände begrenzen den Permeatraum, durch den die Hohlfasern gespannt sind. An eine Öffnung des Permeatraums schließt sich eine Pumpe an. Bei der Entgasung ist es eine Gaspumpe, die das Gas von den Hohlfasern wegführt, somit aus deren Sicht eine Vakuumpumpe. In Figur 7 zur Begasung ist es eine Gaspumpe, die das Gas zu den Hohlfasern leitet und im Permeatraum einen Überdruck erzeugt. Die Benutzung von Hohlfaserkontaktoren hat insbesondere den Vorteil, dass eine optimale Versorgung der Mikroorganismen mit Gas erfolgt, da mit ihrer Hilfe das Gas am besten im Medium gelöst werden kann, wodurch eine bessere und energiesparsamere Versorgung als durch herkömmliche Methoden wie Besprudelung und Vermischung erreicht wird. Wir haben gefunden, dass durch den Einsatz von Hohlfasern insbesondere der Sauerstoffgehalt unter 3 % reduziert werden kann.

In einer bevorzugten Ausführungsform des erfindungsgemäßen Verfahrens kommen im ersten Abschnitt als PTM Cyanobakterien insbesondere Gloeothece 6909, Plectonema boryanum, Anabaena sp. und Nostoc sp. vor. Es zeigt sich, dass Cyanobakterien sich sehr gut für das erfindungsgemäße Verfahren eignen. Im Gegensatz zu Grünalgen, die in der sogenannten Grünlücke nicht absorbieren, können sie das Licht aller Wellenlängen mit Hilfe von Phycobillinen absorbieren und in chemische Energie umwandeln (MacColl R. 1998). Die Effizienz der Lichtverwertung ist bei dem Phycobillin Phycoerythrin sogar höher als bei Chlorophyll. Dadurch können Cyanobakterien auch Schwachlichtbereiche erfolgreich besiedeln, wie man sie beispielsweise auf der Unterseite von Flussgeröll oder in tiefen Schichten von Seen findet (Sari S. 2010). Folglich können sie im Produktionsmodus organisches Material auch bei geringen Lichtintensitäten herstellen. Des Weiteren werden Cyanobakterien bevorzugt, weil sie licht- und temperaturrobust sind, d.h. höhere Bestrahlungen und niedrigere Temperaturen ohne größere Beschädigungen aushalten (Latifi A. et al. 2009). Sie gehören zu den ältesten Lebewesen und haben beinahe jeden Lebensraum der Erde besiedelt. Im Gegensatz zu eukaryotischen PTM verfügen sie über keine vergleichbaren Zellkompartimente, so dass sie verhältnismäßig einfach zu analysieren und zu manipulieren sind. Nicht zuletzt sind sie für das erfindungsgemäße Verfahren aufgrund ihrer hohen Produktionsraten von organischem Material von Vorteil. Die genannten Arten und Gattungen zeichnen sich beispielsweise durch hohe Produktions- und Sekretionsraten von Glycolsäure aus (Renström E. et al. 1989). Weiter bevorzugt werden biofilmbildende Cyanobakterien. Die genannten Arten und Gattungen bilden im ersten Abschnitt besagten Biofilm und werden dadurch nicht in den zweiten Abschnitt transportiert. Weiter bevorzugt werden geringfügig das produzierte organische Material verstoffwechselnde Cyanobakterien. Eine Strategie der Cyanobakterien, mit produziertem organischem Material zu verfahren, ist die Sekretion in das umliegende Milieu anstelle des energieintensiven Umbaus in für sie verwertbare Zwischenprodukte. Bevorzugt werden Arten, die nicht mehr als 10 % des organischen Materials verstoffwechseln. Viele Grünalgenarten wie Chlamydomomas reinhardtii produzieren zwar in nicht unerheblichen Maße Glycolsäure. Jedoch verstoffwechseln sie diese zu einem großen Teil (Moroney J.V. et al. 1986), weswegen sie für das erfindungsgemäße Verfahren weniger gut geeignet sind. Durch das erfindungsgemäße Verfahren kann im Ergebnis eine gentechnikfreie auch beim Verbraucher einsetzbare Technologie verwendet werden.

In einer bevorzugten Ausführungsform des erfindungsgemäßen Verfahrens sind die Methanbildner im zweiten Abschnitt eine Mischung aus acetotrophen und hydrogenotrophen Archaea. Die acetotrophen Archaea insbesondere Methanosarcina sp. oder Synthrophobotulus sp. spalten das organische Material aus dem ersten Abschnitt in Kohlenstoffdioxid und Wasserstoff auf. Es wird im Anschluss daran von hydrogenotrophen Archaea insbesondere Methanocella paludicola, Methanocella arvoryzae oder Methanopyrus kandleri zur Biomethanherstellung genutzt. Weiterhin bevorzugt werden im zweiten Abschnitt Mischkulturen verwendet, die durch Selektion aus Sedimenten von Seen und Meeren, aus Rinderpansen, dem Darm von Termiten und anderen Tieren, Reisfeldern, Sümpfen oder Biogasanlagen gewonnen wurden. Bei der Selektion wird den Archaea bis zur ausschließlichen Kohlenstoffversorgung mittels organischen Materials jenes stufenweise bevorzugt in dem zuvor beschriebenen Turbidostaten zugegeben. Dazu wurde durch die Erfinder Klärschlamm aus einer konventionellen Biogasanlage verwendet.

In einer bevorzugten Ausführungsform des erfindungsgemäßen Verfahrens sind Hemmstoffe des intrazellulären Abbaus des organischen Materials und der intrazellulären Kohlenstoffdioxidspeicherung im ersten Abschnitt vorhanden. Hemmstoffe des intrazellulären Abbaus von Glycolsäure sind zum Beispiel 3-Decyl-2,5-dioxo-4-hydroxy-3-pyrrolin (Stenberg K. 1997), 4-Carboxy-5-(1-pentyl)hexylsulfanyl-1,2,3-triazol (Stenberg K. 1997), Butyl 2-hydroxy-3-butynoate (Doravari S. et al. 1980) und α-Hydroxy-2-pyridinmethansulfonsäure (Zelitch I. 1966), welche vor allem die Glycolsäureoxidase hemmen. Hemmstoffe der Kohlenstoffdioxidspeicherung sind unter anderem Acetazolamid (Moroney J.V. et al. 2001) und Glycolaldehyd (Miller A.G. et al. 1989), welche die Konversion von Kohlenstoffdioxid zu Hydrogencarbonat, einer Speicherform des Kohlenstoffdioxids, unterdrücken. Die Hemmstoffe werden bevorzugt dem Medium im ersten Abschnitt zur Optimierung der Produktion organischen Materials beigefügt.

In einer bevorzugten Ausführungsform des erfindungsgemäßen Verfahrens wird in den PTM die Expression und/oder die Aktivität der Glycolsäuredehydrogenase (EC 1.1.99.14) und/oder Glycolsäureoxidase (EC 1.1.3.15) unterdrückt oder es werden anhand von Aktivitätsmessungen entsprechende PTM selektiert. Die Unterdrückung geschieht zum Beispiel durch shRNA (small hairpin RNA), ein RNA-Molekül mit einer Haarnadelstruktur, dass künstlich Gene durch RNA-Interferenz (RNAi) stilllegen kann. Alternativ können siRNAs (small interfering RNA), kurze, einzel- oder doppelsträngige Ribonukleinsäuremoleküle mit einer Länge von 20 bis 25 Basenpaaren genutzt werden. Sie fangen komplementäre einzelsträngige RNA-Moleküle ab und unterbinden damit die Expression des dazugehörigen Proteins. Bevorzugt wird weiterhin die Kohlenstoffdioxidanreicherung durch Carboxysomen und Pyrenoide gehemmt. Dazu wird die Expression der in den Carboxysomen für die Kohlenstoffdioxidanreicherung notwendigen Carboanhydrase (E.C. 4.2.1.1.) durch shRNA, siRNA oder andere Methoden wie Genknockouts unterdrückt. Bevorzugt werden die Ribulose-1,5-bisphosphat-Carboxylase/Oxygenase (EC 4.1.1.39) und/oder Glycolsäurephosphat-Phosphatase (EC 3.1.3.18) überexprimiert. Die Überexpression kann durch Einführung eines Plasmids mit dem jeweiligen Gen in den Organismus oder durch gegebenenfalls mehrfachen Einbau einer Überexpressionskassette mit dem jeweiligen Gen in das Genom des Organismus erfolgen, wobei das Gen jeweils von einem starken Promoter flankiert wird. RuBisCO ist innerhalb des Calvinzyklus das für die Kohlenstoffdioxid-/Sauerstoffassimilation bestimmende Enzym, während die Glycolsäurephosphat-Phosphatase der Herstellung der sekretierten Glycolsäure vorausgeht (Figur 10). Die Expression beider Enzyme führt daher zu einer verstärkten Produktion der Glycolsäure. Weiter bevorzugt wird die RuBisCO vom Typ II in den PTM exprimiert. Die Expression kann durch Einführung eines Plasmids oder einer Kassette in das Genom erfolgen. RuBisCO vom Typ II, die bakteriellen Ursprungs ist, hat eine höhere Wechselzahl als RuBisCO vom Typ I, so dass ein Einbau für eine höhere Produktivität von Vorteil ist. Bevorzugt wird die Ausscheidung organischen Materials insbesondere von Glycolsäure durch die Zellmembran verstärkt. Dies geschieht durch die Überexpression oder die verstärkte Aktivierung von Transporterproteinen in der Zellmembran, die für den Transport des jeweiligen organischen Materials zuständig sind. Bei den vorteilhaft zu benutzenden Cyanobakterien ist keine Zielsteuerung der Transporter in kompartimentäre Membranen nötig, da Prokaryoten über keine den anderen PTM entsprechenden Kompartimente verfügen, während es bei den eukaryotischen PTM nötig ist.

Die erfindungsgemäße Vorrichtung zur Herstellung von Biogas in einem kombinierten Fermenter umfasst einen ersten Abschnitt, der teilweise lichtdurchlässig ist, mit phototrophen Mikroorganismen, die sich in einem Medium befinden. Die Lichtdurchlässigkeit wird durch die Verwendung von transparenten Materialien wie Glas, Formen von Acrylglas (Polymethylmetacrylate), Polycarbonat, Polyvinylchlorid, Polystyrol, Polyphenylenether und Polyethylen gewährleistet. Der erste Abschnitt ist bevorzugt als ein flaches Modul vergleichbar einem Solarpanel ausgestaltet, da innerhalb der Achse entlang der Sonneneinstrahlung die Tiefe des ersten Abschnitts durch die maximale Beleuchtungstiefe limitiert ist. Erfindungsgemäß sind die PTM derart immobilisiert, dass mehr als 90 % der einfallenden Sonnenstrahlung absorbiert wird, wobei eine Dicke von nicht mehr als 10 mm nicht überschritten wird. Die Module eignen sich zur einfachen Installation auf Freiflächen oder Häuserdächern und können beliebig miteinander kombiniert und erweitert werden. Nach dem Erreichen der Lebensdauer der PTM können diese ausgetauscht werden und herkömmlichen Biogasanlagen als ertragssteigernde Beimischung zugeführt werden.

Die Vorrichtung umfasst weiterhin einen zweiten Abschnitt, der lichtundurchlässig ist, mit Methanbildnern, die sich in dem Medium befinden, das im Austauschmodus mit dem ersten Abschnitt ausgetauscht wird, wobei der Sauerstoffgehalt reduziert wird. Weiterhin Bestandteil ist ein Verbindungssystem zwischen erstem Abschnitt und zweitem Abschnitt, in dem der Austausch und die Be- und Entgasung des Mediums mit Hilfe von Filtern stattfindet, wobei die Filter mit Gaszu- und Gasableitungen verbunden sind. Ein weiterer Bestandteil ist eine Biogasableitung vom zweiten Abschnitt zur Ableitung des entstehenden Biogases. Pumpen und Ventile dienen der Verteilung der Flüssigkeits- und Gasströme entsprechend der beispielhaft anhand der Zeichnungen erläuterten Ausführungsform. Die weitere Ausgestaltung der Vorrichtung richtet sich nach den zuvor beschriebenen vorteilhaften Verfahren. Bevorzugt sind beide Abschnitte mit Behältern, die Nährstoffe wie Spurenelemente beinhalten oder der Abfuhr von Nebenprodukten dienen, verbunden. Die Materialien der beiden Abschnitte abgesehen von dem transparenten Bereich im ersten Abschnitt sind entsprechend der gestellten Ansprüche zum Beispiel Metalle oder Kunststoffe.

In einer bevorzugten Ausführungsform der erfindungsgemäßen Vorrichtung umfasst das Verbindungssystem ein erstes und zweites Verbindungsrohr zur möglichen Etablierung eines Ganzreaktorkreislaufs im Austauschmodus. Weiterhin umfasst das Verbindungssystem vorzugsweise ein Querverbindungsrohr, das das erste Verbindungsrohr und das zweite Verbindungsrohr miteinander verbindet. Dadurch kann ein Teilreaktorkreislauf für die Übergänge zwischen Regenerations- und Produktionsmodus etabliert werden. Weiterhin bevorzugt sind Filter als Teilstücke der Verbindungsrohre zwischen den Abschnitten und dem Querverbindungsrohr vorgesehen. Diese Filter dienen der Be- und Entgasung im Austauschmodus (erster bis vierter Filter) und in den Übergängen vom Austauschmodus zum Regenerationsmodus und vom Regenerationsmodus zum Produktionsmodus (erster und zweiter Filter). Weiterhin umfasst die Vorrichtung vorzugsweise ein zweites Querverbindungsrohr, das den ersten Abschnitt und das Querverbindungsrohr verbindet, und einen fünften Filter als Teilstück des zweiten Querverbindungsrohrs mit einer Luftleitung mit Luftfilter bevorzugt mit einer Porengröße von 0,2 µm oder kleiner zur Entkeimung der eingesaugten Luft. Der fünfte Filter dient der Be- und Entgasung mit Luft in den Übergängen vom Austauschmodus zum Regenerationsmodus und vom Regenerationsmodus zum Produktionsmodus, wobei der erste und zweite Filter ihre jeweilige Be- bzw. Entgasungsfunktion mit Sauerstoff wahrnehmen. Bevorzugt verfügt der Fermenter über eine Sauerstoffableitung, eine Sauerstoffzuleitung, eine Ersatzgasableitung und eine Ersatzgaszuleitung, jeweils verbunden mit einer Gaspumpe und dem jeweiligen Filter sowie einen Ersatzgasspeicher und einem Sauerstoffspeicher, jeweils verbunden mit den Ersatzgas- bzw. Sauerstoffleitungen. Dadurch können die Gase nach kombinierten Be- und Entgasungsvorgängen zwischengespeichert werden. Weiterhin bevorzugt geht die Biogasableitung vom zweiten Abschnitt in einen Biogasfilter und anschließend in einen Biomethanspeichertank über. Vorzugsweise ist der Biogasfilter mit dem Ersatzgasspeicher verbunden. Dadurch kann das durch den Biogasfilter abgezweigte Kohlenstoffdioxid dem Ersatzgaskreislauf wieder zur Verfügung gestellt werden. Auch eine Bereitstellung überflüssigen Kohlenstoffdioxids im Regenerationsmodus ist denkbar. Es handelt sich im Ergebnis um einen doppelten Kreislauf. Ein pH-Sensor im ersten Abschnitt dient vorzugsweise der Feststellung wie hoch der Anteil organischer Säure im Medium ist und wann entsprechend der Austauschmodus einzuleiten ist. Alternativ können Solarsensoren zur Feststellung der Aktivität der PTM eingesetzt werden.

In einer bevorzugten Ausführungsform der erfindungsgemäßen Vorrichtung wird der erste Abschnitt vor zu starker Sonneneinstrahlung geschützt. Dies dient dem Schutz der PTM, deren Photosysteme bei zu starker Sonneneinstrahlung beschädigt werden können und/oder die durch die Freisetzung schädlicher reaktiver Sauerstoffspezies gefährdet sind. Insbesondere kann der lichtdurchlässige Bereich des ersten Abschnitts schrittweise verdunkelt werden. Beispielsweise können im lichtdurchlässigen Bereich des ersten Abschnitts elektrochrome Materalien Verwendung finden.

In einer bevorzugten Ausführungsform der erfindungsgemäßen Vorrichtung sind die phototrophen Mikroorganismen bevorzugt auf Zellulose oder einem beweglichen Trägermaterial insbesondere in gasdurchlässigen Gelkapseln und die Methanbildner bevorzugt auf Aktivkohle und/oder einem beweglichem Trägermaterial insbesondere in gasdurchlässigen Gelkapseln immobilisiert. Die Immobilisierung dient dazu, die PTM und die Methanbildner in den jeweils für sie vorgesehenen Abschnitten zu fixieren. Nur das Medium soll ausgetauscht werden. Immobilisierungsverfahren wie (kovalente) Bindung an die Oberfläche, Ausfüllen von Poren, Quervernetzung, Membranabtrennung sowie Einschlussimmobilisierung sind möglich. Beispielsweise können die vorzugsweise biofilmbildenden PTM auf einer Trägeroberfläche wie Zellulose, Alginat, Chitosan oder Agar aufwachsen. Auch bewegliche Träger sind denkbar, die am Ausgang des Abschnitts durch einen Filter zurückgehalten werden und dadurch im jeweiligen Abschnitt zu einer Immobilisierung beitragen. Weiterhin kommen sogenannte Immobilisierungstags in Betracht, d.h. Proteine, die rekombinant auf der Oberfläche der Mikroorganismen exprimiert werden und dort für eine Bindung an einem Trägermaterial sorgen. Die Methanbildner können auch vorteilhaft auf Aktivkohle immobilisiert werden.

Aus der Beschreibung des erfindungsgemäßen Verfahrens und der erfindungsgemäßen Vorrichtung ergeben sich vorteilhafte Wirkungen der Erfindung gegenüber dem Stand der Technik und auch die gewerbliche Anwendbarkeit.

Die vorteilhaften Wirkungen der Erfindung gegenüber dem Stand der Technik schließen eine optimale, vor allem energiesparsame Übertragung des organischen Materials von einem aeroben Abschnitt des kombinierten Fermenters zu einem anaeroben Abschnitt ein. Dabei wird eine verbesserte Belüftung der Mikroorganismen ohne unnötigen Gasverbrauch erreicht. Zusätzlich besteht in einer bevorzugten Variante die Möglichkeit Kohlenstoffdioxid (und Stickstoff) aus der Luft zu assimilieren und damit einen klima- bzw. CO2-neutralen Vorgang einzuleiten, der in der Kohlenstoffdioxidfreisetzung bei der Biomethanverbrennung mündet. Im Ergebnis ist das erfindungsgemäße Verfahren in der dazu vorgesehenen Vorrichtung vor allem im Vergleich zur Herstellung von Biogas aus Pflanzen effektiver, was nicht nur die geringere Ackerflächennutzung und die hohen Kosten bei der Gewinnung der Biomasse betrifft:

| | Biogasertrag m³/t Biomasse (organ. Mat.) | Methangehalt in % |
|---|---|---|
| Mais | 198 | 54 |
| Gras | 158 | 52,9 |
| Euk. PTM | 400 (0,24 ml/mg Glyks.-5/3·10³) | 37,5 |

| | | |
|---|---|---|
| Konv. Biogas-Zahlen aus Schwab M. 2007 | | |

Beschreibung der Zeichnungen
Figur 1 zeigt eine zweidimensionale Ansicht einer erfindungsgemäßen Vorrichtung zur Durchführung des Verfahrens, wobei gestrichelte Stellen einen Ebenenübergang darstellen.
Figuren 2-6 zeigen Ansichten des in Figur 1 dargestellten Fermenters in mehreren Modi.
Figur 7 zeigt den Aufbau eines vorzugsweise als Filter einzusetzenden Hohlfaserkontaktors.
Figur 8 zeigt schematisch den Ablauf des Calvinzyklus in phototrophen Mikroorganismen unter kohlenstoffdioxidreichen Bedingungen (linke Seite) und unter sauerstoffreichen Bedingungen (rechte Seite).
Figur 9 zeigt die durch RuBisCO katalysierten Reaktionen mit den beteiligten Molekülen in Strukturformeln.
Figur 10 zeigt die Oxygenasereaktion der RuBisCO und die weitere Verstoffwechslung der dadurch entstandenen Phospho-Glycolsäure mit den beteiligten Molekülen in Strukturformeln und den Enzymen.
Figur 11 zeigt ein Diagramm zur Glycolsäureproduktion in µmol pro mg Chlorophyll a durch Gloeothece 6909 bei unterschiedlichem Sauerstoff-/Kohlenstoffdioxidverhältnis und unterschiedlichen Bestrahlungsstärken.

### Beschreibung eines Ausführungsbeispiels

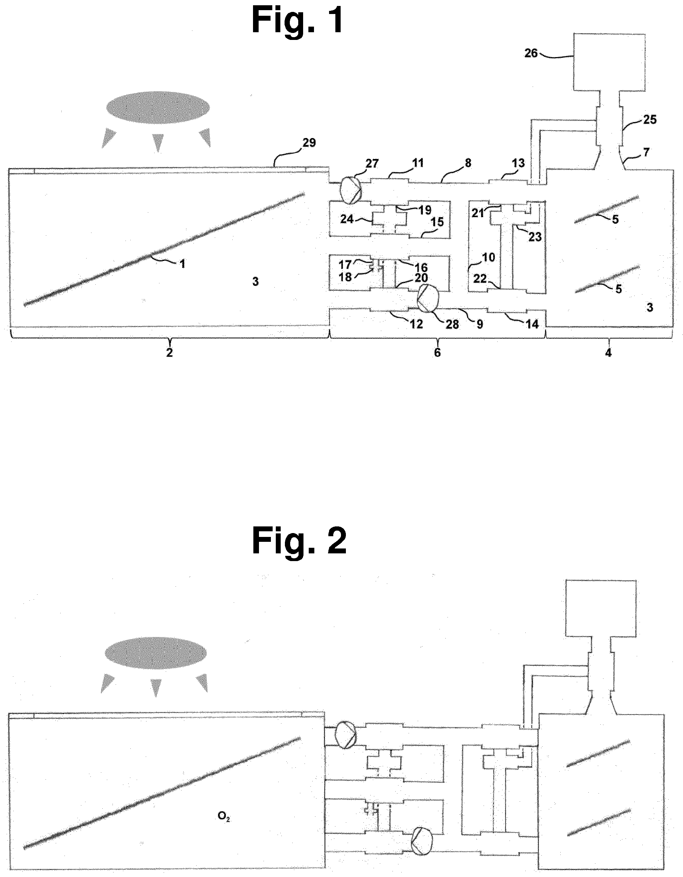

In dem in Figur 2 dargestellten Produktionsmodus produzieren die phototrophen Mikroorganismen (1) Gloeothece 6909, die zuvor angezogen und auf einer Oberfläche immobilisiert wurden sind, unter oxischen Bedingungen und bei Sonnenlichteinstrahlung Glycolsäure, die sie in das Medium (3) sekretieren (Figuren 9 und 11). In diesem Modus sind alle Ventile des ersten Abschnitts (2) und des zweiten Abschnitts (4) zum Verbindungssystem (6) geschlossen. Bei direkter Sonnenbestrahlung im Hochsommer beträgt die Dauer des Produktionsmodus eine Stunde. Eine steuernde Uhr beendet dementsprechend den Produktionsmodus.

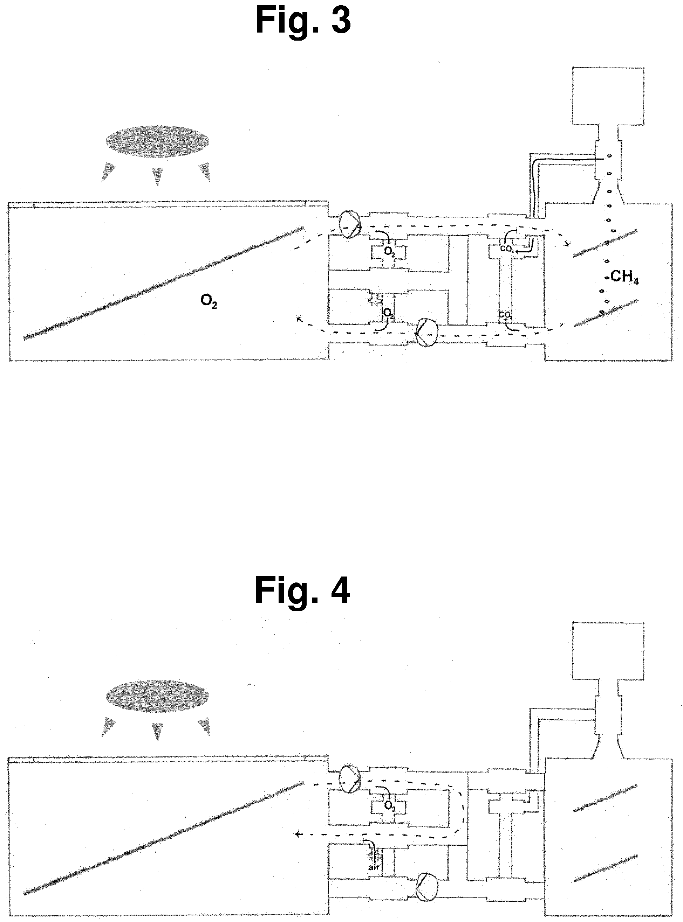

In dem in Figur 3 dargestellten Austauschmodus werden die Ventile zum ersten Verbindungsrohr (8) und zum zweiten Verbindungsrohr (9) geöffnet, während die Ventile zum ersten Querverbindungsrohr (10) und zum zweiten Querverbindungsrohr (15) geschlossen sind. Die erste Pumpe (27) und die zweite Pumpe (28), welche als Schlauchquetschpumpen ausgestaltet sind, pumpen dann das Medium (3) aus dem ersten Abschnitt (2) in den zweiten Abschnitt (4). Dabei passiert das Medium den ersten Filter (11), der in dieser Ausführungsform wie die anderen Filter auch als Hohlfaserkontaktor ausgestaltet ist (Figur 7). Eine Gaspumpe (30) in Form einer Vakuumpumpe stellt in dem Permeatraum (31) zwischen den Trennwänden (32) und den Hohlfasern (33), die vom Medium (3) vom Eingangsraum (34) zum Ausgangsraum (35) durchflossen werden, ein Vakuum her. Die Sauerstoffmoleküle verlassen die Flüssigkeit und penetrieren durch die Hohlfasern in den Permeatraum (31). Von dort werden sie durch die Gaspumpe (30) abgepumpt und durch ein Rohr zum Sauerstoffspeicher (24) und von dort zum zweiten Filter (12) transportiert. Währenddessen verlässt das entgaste Medium den ersten Filter (11) und gelangt durch das erste Verbindungsrohr (8) zum dritten Filter (13), wo eine Gaspumpe (30) einen Überdruck im Permeatraum an Kohlenstoffdioxid erzeugt. Dadurch gelangen die Kohlenstoffdioxidmoleküle aus dem Ersatzgasspeicher (23) durch die Hohlfasern in das Medium (3). Das Medium (3) fließt in der Folge in den zweiten Abschnitt (4). Dort verbrauchen Methanbildner (5), die zuvor auf Glycolsäureverwertung selektiert wurden und immobilisiert sind, die Glycolsäure und wandeln sie in ein Gasgemisch bestehend aus Biomethan und Kohlenstoffdioxid in einem Verhältnis von 3 zu 5 um, welches in Bläschen nach oben sprudelt. Eine Biogasableitung (7) sammelt den Gasstrom und leitet ihn durch einen Biogasfilter (25), einen Hohlfaserkontaktor für die Trennung von Gasen, in dem das Kohlenstoffdioxid abgetrennt wird und in den Ersatzgasspeicher (23) geleitet wird. Das aufgereinigte Biomethan wird hingegen im Biomethanspeichertank (26) gespeichert. Das Medium (3) verlässt den zweiten Abschnitt (4) und wird im vierten Filter (14) von Kohlenstoffdioxid entgast, welches wiederrum der Begasung durch den dritten Filter (13) bzw. der Speicherung in dem Ersatzgasspeicher (23) zur Verfügung gestellt wird. Durch den zweiten Filter (12) wird das Medium (3) wieder mit Sauerstoff begast, der aus der Entgasung von dem ersten Filter (11) stammt. Das Medium (3), welches nun reoxygenisiert ist, gelangt zurück in den ersten Abschnitt (2). Sobald das Medium (3) zwischen den Abschnitten ausgetauscht wurde, beginnt ein neuer Produktionsmodus. Nach mindestens zwei Durchläufen des Produktions- und Austauschmodus beginnt der Übergang zum Regenerationsmodus.

Im Übergang zum Regenerationsmodus sind die Ventile vom zweiten Abschnitt (4), einem Teil des ersten Verbindungsrohrs (8), vom zweiten Verbindungsrohr (9) und einem Teil des ersten Querverbindungsrohrs (10) entsprechend der Figur 4 geschlossen. Die erste Pumpe (27) pumpt das Medium (3) in einem Kreislauf durch den ersten Teil des ersten Verbindungsrohrs (8), den oberen Teil des ersten Querverbindungsrohrs (10) und das zweite Querverbindungsrohr (15). Dabei passiert das Medium (3) zunächst den ersten Filter (11), in dem es von Sauerstoff entgast wird, der in dem Sauerstoffspeicher (24) zwischengespeichert wird. Im Anschluss daran fließt das Medium (3) durch den fünften Filter (16) und wird dort mit Umgebungsluft, die durch eine Luftleitung (17) sowie durch einen Luftfilter (18) angesaugt wird, begast. Das Medium gelangt schließlich zurück in den ersten Abschnitt (2).

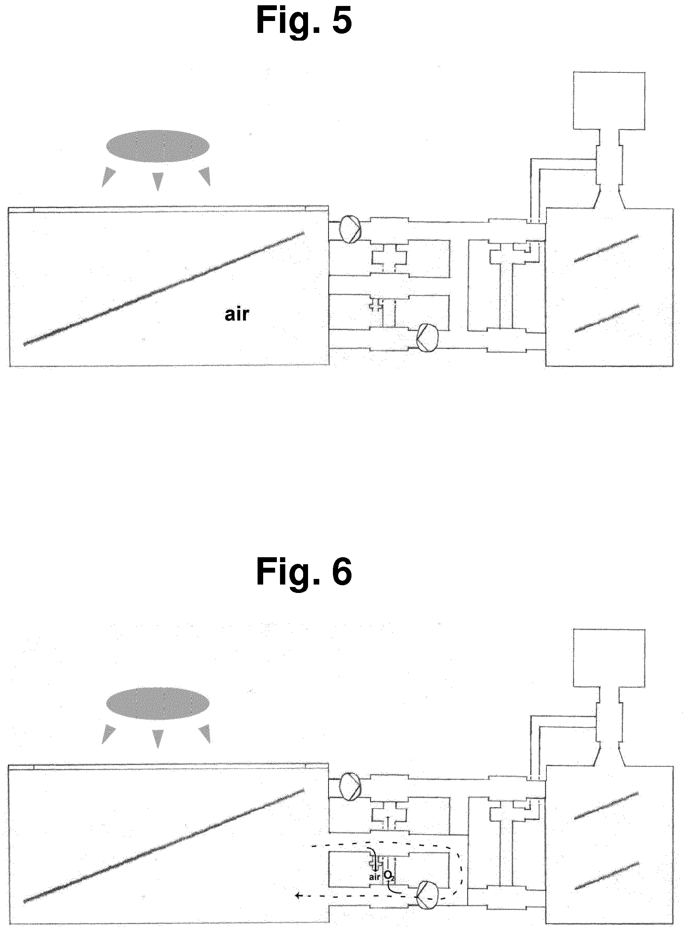

Im Regenerationsmodus sind die Ventile der Abschnitte zum Verbindungssystem (6) wie in Figur 5 gezeigt geschlossen. Bei Sonnenlichtbestrahlung wird durch die PTM Kohlenstoff (Figur 8) und Stickstoff aus der im Medium (3) gelösten Luft assimiliert. Die Dauer des Regenerationsmodus richtet sich nach der Dauer eines Durchlaufs des Produktionsmodus.

Im Übergang zum Produktionsmodus sind die Ventile vom zweiten Abschnitt (4), erstem Verbindungsrohr (8), einem Teil des zweiten Verbindungsrohrs (9) und einem Teil des ersten Querverbindungsrohrs (10) entsprechend der Figur 6 geschlossen. Die zweite Pumpe (28) pumpt das Medium (3) in einem Kreislauf durch den ersten Teil des zweiten Verbindungsrohrs (9), den unteren Teil des ersten Querverbindungsrohrs (10) und das zweite Querverbindungsrohr (15). Dabei passiert das Medium (3) zunächst den fünften Filter (16), in dem es von der restlichen Luft entgast wird, die durch die Luftleitung (17) sowie durch den Luftfilter (18) in die Umgebung abgegeben wird. Im Anschluss daran fließt das Medium (3) durch den zweiten Filter (12) und wird dort mit dem zuvor gespeicherten Sauerstoff aus dem Sauerstoffspeicher (24) begast. Es gelangt schließlich in den ersten Abschnitt (2) zurück. Die Ventile des ersten Abschnitts (2) werden geschlossen. Der Produktionsmodus startet bei Sonnenlichtbestrahlung, die durch einen Solardetektor registriert wird.

Es wurde folgende Nichtpatentliteratur zitiert:
Barber J. 2009 - Photosynthetic energy conversion: natural and artificial, Chemical Society Reviews 2009 (38), S. 185-196
Bauwe H. 2011 - Photorespiration: The Bridge to C4 Photosynthesis. In: Agepati S. Raghavendra (Hrsg.) und Rowan F. Sage (Hrsg.): C4 Photosynthesis and Related CO2 Concentrated Mechanisms (Advances in Photosynthesis and Respiration), Springer Netherlands 2011, S. 81-108
Bericht des FHI 2011 - Algen - nachhaltige Rohstoffquelle für Wertstoffe und Energie, Fraunhoferinstitut für Grenzflächen- und Bioverfahrenstechnik 2011, S. 2
Bernacchi S. et al. 2014 - Process efficiency simulation for key process parameters in biological methanogenesis, AIMS Bioeng. 2014 (1), S. 53-71
Blankenship R.E. et al. 2011 - Comparing Photosynthetic and Photovoltaic Efficiencies and Recognizing the Potential for Improvement, Science 2011 (332), S. 805-809
Carrieri D. et al. 2015 - Enhancing photo-catalytic production of organic acids in the cyanobacterium Synechocystis sp. PCC 6803 ΔglgC, a strain incapable of glycogen storage, Microb. Biotechnol. 2015 (8), S. 275-280
Dismukes G.C. et al. 2008 - Aquatic Phototrophs: efficient alternatives to land-based crops for fuels, Current Opinion in Biotechnology 2008 (19), S. 235-240
Doravari S. et al. 1980 - Effect of butyl 2-hydroxy-3-butynoate on sunflower leaf photosynthesis and photorespiration, Plant Physiol. 1980 (66), S. 628-631
Edenborn H.M. et al. 1985 - Glycolate metabolism by Pseudomonas sp., strain S227, isolated from a coastal marine sediment, Marine Biology (88), S. 199-205
Egli C. et al. 1989 - Monochloro- and dichloroacetic acids as carbon and energy sources for a stable, methanogenic mixed culture, Arch. Microbiol. 1989 (152), S. 218-223
Fachverband Biogas Prognose - Branchenzahlenprognose für die Jahre 2014 und 2015, Fachverband Biogas e.V., Juni 2014, S. 2
Friedrich M. et al. 1991 - Fermentative degradation of glycolic acid by defined syntrophic cocultures, Arch. of Microbiol. 1991 (156), S. 398-404
Friedrich M. et al. 1996 - Phylogenetic Positions of Desulfofustis glycolicus gen. nov., sp. nov., and Synthrobotulus glycolicus gen. nov., sp. nov., Two New Strict Anaerobes Growing with Glycolic Acid, Int. J. of Sys. Bact., 1996, S. 1065-1069
Günther A. et al. 2012 - Methane production from glycolate excreting algae as a new concept in the production of biofuels, Biores. Tech. 2012 (121), S. 454-457
Kurz W.G.W, et al. 1973 - Metabolism of glycolic acid by Azotobacter chroococcum PRL H62, Can. J. of Microbiol. 1973 (19), S. 321-324
Latifi A. et al. 2009 - Oxidative stress in cyanobacteria, FEMS Microbiol. Rev. 2009 (33), S. 258-278
Linder H. 1998 - Biologie, Schroedel, 21. Aufl. 1998, S. 43
Lorimer G.H. 1981 - The carboxylation and oxygenation of ribulose 1,5-bisphosphate; The primary events in photosynthesis and photorespiration. Ann. Rev. Plant Physiol. 1981 (32), S. 349-383
MacColl R. 1998 - Cyanobacterial phycobilisomes, J. Struct. Biol. 1998 (15), S. 311-334 Masukawa H. et al. 2012 - Genetic Engineering of Cyanobacteria to Enhance Biohydrogen Production from Sunlight and Water, AMBIO 2012 (41), S. 169-173
Miller A.G. et al. 1989 - Glycolaldehyde Inhibits CO2 Fixation in the Cyanobacterium Synechococcus UTEX 625 without Inhibiting the Accumulation of Inorganic Carbon or the Associated Quenching of Chlorophyll a Fluorescence, Plant Phys. 1989 (91), S. 1044-1049
Moroney J. V. et al. 1986 - Glycolate metabolism and excretion by Chlamydomonas reinhardtii, Plant Physiol. 1986 (82), S. 821-826
Moroney J.V. et al. 2001 - Carbonic anhydrases in plants and algae, Plant, Cell & Env. 2001 (24), S. 141-153
Nabors M.W. et al. 2007 - Botanik, Pearson Studium, 1. Aufl. 2007, S. 221-222
Renström E. et al. 1989 - Glycolate metabolism in cyanobacteria. I. Glycolate excretion and phosphoglycolate phosphatase activity, Phys. Plant. 1989 (43), S. 137-143
Sari, S. 2010 - Untersuchung der Dehydrogenierung von NADH/NADPH in isolierten und gereinigten Membranen von drei verschiedenen Cyanobakterienspezies, die in zwei verschiedenen Bedingungen gezüchtet wurden, Diplomarbeit, Universität Wien 2010, S. 8
Schwab M. 2007 - Biogaserträge aus Energiepflanzen - Eine kritische Bewertung des Datenpotentials, Kuratorium für Technik und Bauwesen in der Landwirtschaft e. V. 2007, S. 4
Stein J. 1973 - Handbook of Phycological methods. Culture methods and growth measurements. Cambr. Univ. Press. 1973, S. 448ff.
Stenberg K. 1997 - Three-dimensional structures of glycolate oxidase with bound active-site inhibitors, Prot. Sc. 1997 (6), S. 1009-1015
Zelitch I. 1966 - Increased Rate of Net Photosynthetic Carbon Dioxide Uptake Caused by the Inhibition of Glycolate Oxidase, Plant Physiol. 1966 (41), S. 1623-1631

## Patentansprüche

1. Verfahren zur Herstellung von Biogas in einem kombinierten Fermenter, in dem phototrophe Mikroorganismen (1) in einem ersten Abschnitt (2) organisches Material insbesondere Glykolsäure aus Kohlenstoffdioxid und Sauerstoff herstellen und in ein Medium (3) sekretieren (Produktionsmodus), das in einen zweiten Abschnitt (4) geleitet wird, in dem Methanbildner (5) unter anoxischen Bedingungen daraus Biomethan und Kohlenstoffdioxid herstellen, **dadurch gekennzeichnet, dass** das Medium (3) beim Übergang vom ersten Abschnitt (2) zum zweiten Abschnitt (4) von Sauerstoff entgast und bei einem Rücklauf mit Sauerstoff wieder begast wird (Austauschmodus).

2. Verfahren nach Anspruch 1, **dadurch gekennzeichnet, dass**, während der Kontakt zum zweiten Abschnitt (4) unterbrochen ist,
- das Medium (3) von Sauerstoff entgast und bei einem Rücklauf mit Luft aus der Umgebung begast wird;
- danach die Luft von den phototrophen Mikroorganismen (1) zur Kohlenstoffassimilation genutzt wird (Regenerationsmodus);
- danach das Medium (3) von Luft entgast und bei einem Rücklauf mit Sauerstoff begast wird.

3. Verfahren nach Anspruch 1 oder 2, **dadurch gekennzeichnet, dass** das Verhältnis von Kohlenstoffdioxid zu Sauerstoff in dem im ersten Abschnitt (2) befindlichen Medium (3) im Produktionsmodus gering, bevorzugt 1:800 bis 1:3000 ist.

4. Verfahren nach einem der vorhergehenden Ansprüche, **dadurch gekennzeichnet, dass** im Austauschmodus das Medium (3) beim Übergang vom ersten Abschnitt (2) zum zweiten Abschnitt (4) nach der Sauerstoffentgasung mit einem Ersatzgas insbesondere mit Kohlenstoffdioxid oder Stickstoff begast wird und beim Rücklauf vor der Sauerstoffbegasung von dem Ersatzgas wieder entgast wird.

5. Verfahren nach einem der vorhergehenden Ansprüche, **dadurch gekennzeichnet, dass** von dem im zweiten Abschnitt (4) entstehenden Biogas das Kohlenstoffdioxid abgetrennt und zur Kohlenstoffdioxidbegasung nach Anspruch 4 genutzt wird.

6. Verfahren nach einem der vorhergehenden Ansprüche, **dadurch gekennzeichnet, dass** zur Be- und Entgasung Filter insbesondere Hohlfaserkontaktoren verwendet werden, wobei die Porengröße unter 0,1 µm bevorzugt unter 0,04 µm beträgt.

7. Verfahren nach einem der vorhergehenden Ansprüche, **dadurch gekennzeichnet, dass** im ersten Abschnitt (2) als phototrophe Mikroorganismen (1) bevorzugt biofilmbildende und bevorzugt geringfügig weiter bevorzugt unter 10 % das produzierte organische Material verstoffwechselnde Cyanobakterien insbesondere Gloeothece 6909, Plectonema boryanum, Anabaena sp. und Nostoc sp. vorkommen.

8. Verfahren nach einem der vorhergehenden Ansprüche, **dadurch gekennzeichnet, dass** die Methanbildner (5) im zweiten Abschnitt (4) eine Mischung aus acetotrophen und hydrogenotrophen Archaea sind, wobei die acetotrophen Archaea insbesondere Methanosarcina oder Synthrophobotulus das organische Material aus dem ersten Abschnitt (2) in Kohlenstoffdioxid und Wasserstoff spalten, welches von hydrogenotrophen Archaea insbesondere Methanocella paludicola, Methanocella arvoryzae oder Methanopyrus kandleri zur Biomethanherstellung genutzt wird, und/oder Mischkulturen, die durch Selektion aus Sedimenten von Seen und Meeren, aus Rinderpansen, dem Darm von Termiten und anderen Tieren, Reisfeldern, Sümpfen oder Biogasanlagen gewonnen wurden, sind.

9. Verfahren nach einem der vorhergehenden Ansprüche, **dadurch gekennzeichnet, dass** Hemmstoffe des intrazellulären Abbaus des organischen Materials und/oder der intrazellulären Kohlenstoffdioxidspeicherung im ersten Abschnitt (2) vorhanden sind.

10. Verfahren nach einem der vorhergehenden Ansprüche, **dadurch gekennzeichnet, dass** in den phototrophen Mikroorganismen (1)
- die Expression und/oder die Aktivität der Glykolsäuredehydrogenase und/oder Glykolsäureoxidase unterdrückt wird oder ist;
- die Kohlenstoffdioxidanreicherung durch Carboxysomen und Pyrenoide gehemmt wird;
- die Ribulose-1,5-bisphosphat-Carboxylase/Oxygenase und/oder Glykolsäurephosphat-Phosphatase überexprimiert werden;
- die Ribulose-1,5-bisphosphat-Carboxylase/Oxygenase Typ 11 exprimiert wird;
- die Ausscheidung organischen Materials insbesondere von Glykolsäure durch die Zellmembran verstärkt wird.

11. Vorrichtung zur Herstellung von Biogas in einem kombinierten Fermenter mit folgenden Bestandteilen:
- einen ersten Abschnitt (2), der teilweise lichtdurchlässig ist, mit phototrophen Mikroorganismen (1), die sich in einem Medium (3) befinden;
- einen zweiten Abschnitt (4), der lichtundurchlässig ist, mit Methanbildnern (5), die sich in dem Medium (3) befinden, das im Austauschmodus mit dem ersten Abschnitt (2) ausgetauscht wird, wobei der Sauerstoffgehalt reduziert wird;
- ein Verbindungssystem (6) zwischen erstem Abschnitt (2) und zweitem Abschnitt (4);
- Filter in dem Verbindungsystem (6) zur Be- und Entgasung des Mediums (3), die mit Gaszu- und Gasableitungen verbunden sind,
- eine Biogasableitung (7) vom zweiten Abschnitt (4);
- Pumpen und Ventile zur Verteilung der Flüssigkeits- und Gasströme.

12. Vorrichtung nach Anspruch 11, **dadurch gekennzeichnet, dass**
- das Verbindungssystem (6) ein erstes Verbindungsrohr (8) und ein zweites Verbindungsrohr (9) umfasst;
- das Verbindungssystem (6) ein erstes Querverbindungsrohr (10) umfasst, das das erste Verbindungsrohr (8) und das zweite Verbindungsrohr (9) miteinander verbindet;
- ein erster Filter (11) ein Teilstück des ersten Verbindungsrohrs (8) zwischen dem ersten Abschnitt (2) und dem ersten Querverbindungsrohr (10) ist;
- ein zweiter Filter (12) ein Teilstück des zweiten Verbindungsrohrs (9) zwischen dem ersten Abschnitt (2) und dem ersten Querverbindungsrohr (10) ist;
- ein dritter Filter (13) ein Teilstück des ersten Verbindungsrohrs (8) zwischen dem zweiten Abschnitt (4) und dem ersten Querverbindungsrohr (10) ist;
- ein vierter Filter (14) ein Teilstück des zweiten Verbindungsrohrs (9) zwischen dem zweiten Abschnitt (4) und dem ersten Querverbindungsrohr (10) ist;
- ein zweites Querverbindungsrohr (15), das den ersten Abschnitt (2) und das erste Querverbindungsrohr (10) verbindet;
- ein fünfter Filter (16) ein Teilstück des zweiten Querverbindungsrohrs (15) ist und mit einer Luftleitung (17) mit Luftfilter (18) bevorzugt mit einer Porengröße von 0,2 µm oder kleiner verbunden ist;
- eine Sauerstoffableitung (19), eine Sauerstoffzuleitung (20), eine Ersatzgaszuleitung (21) und eine Ersatzgasableitung (22) vorhanden sind;
- ein Ersatzgasspeicher (23) und ein Sauerstoffspeicher (24) vorhanden sind;
- ein Biogasfilter (25) als Teilstück der Biogasableitung (7) mit einem sich daran anschließenden Biomethanspeichertank (26) vorhanden ist;
- der Ersatzgasspeicher (23) mit dem Biogasfilter (25) verbunden ist;
- eine erste Pumpe (27) als Teilstück des ersten Querverbindungsrohrs (8) zwischen erstem Abschnitt (2) und erstem Filter (11) sowie eine zweite Pumpe (28) als Teilstück des zweiten Verbindungsrohrs (9) zwischen erstem Querverbindungsrohr (10) und zweitem Filter (12) vorhanden ist.

13. Vorrichtung nach einem der vorhergehenden Ansprüche, **dadurch gekennzeichnet, dass** der erste Abschnitt (2) vor zu starker Sonneneinstrahlung geschützt wird, insbesondere der lichtdurchlässige Bereich (29) des ersten Abschnitts (2) schrittweise verdunkelt werden kann.

14. Vorrichtung nach einem der vorhergehenden Ansprüche, **dadurch gekennzeichnet, dass** die phototrophen Mikroorganismen (1) bevorzugt auf Zellulose oder einem beweglichen Trägermaterial insbesondere gasdurchlässigen Gelkapseln und die Methanbildner (5) bevorzugt auf Aktivkohle oder einem beweglichem Trägermaterial insbesonder gasdurchlässigen Gelkapseln immobilisiert sind.

15. Vorrichtung nach einem der vorhergehenden Ansprüche, **dadurch gekennzeichnet, dass** die Filter als Hohlfaserkontaktoren insbesondere mit einer Gaspumpe (30), einem Permeatraum (31), Trennwänden (32), Hohlfasern (33), einem Eingangsraum (34) und einem Ausgangsraum (35) ausgestaltet sind.

## Claims

1. A method for the production of biogas in a combined fermenter, in which phototrophic microorganisms (1) in a first section (2) produce organic material, in particular glycolic acid from carbon dioxide and oxygen, and secrete it into a medium (3) (production mode) that is fed into a second section (4) in which methanogens (5) produce biomethane and carbon dioxide under anoxic conditions, **characterized in that** the medium (3) is degassed by oxygen during the transition from the first section (2) to the second section (4) and is backfilled with oxygen during a return (exchange mode).

2. A method according to claim 1, **characterized in that**, while the contact to the second section (4) is interrupted,
- the medium (3) is degassed by oxygen and is gassed with air from the environment during a return;
- the air is then used by the phototrophic microorganisms (1) for carbon assimilation (regeneration mode);
- the medium (3) is then degassed by air and gassed with oxygen-during a return flow.

3. A method according to claim 1 or 2, **characterized in that** the ratio of carbon dioxide to oxygen in the medium (3) in the production mode located in the first section (2) is low, preferably 1: 800 to 1: 3000.

4. A method according to one of the preceding claims, **characterized in that** in the exchange mode the medium (3) is gassed with a substitute gas after the oxygen degassing during the transition from the first section (2) to the second section (4), in particular with carbon dioxide or nitrogen, and that it is degassed by the substitute gas before the oxygen gassing.

5. A method according to one of the preceding claims, **characterized in that** the carbon dioxide is separated from the biogas produced in the second section (4) and is used for carbon dioxide gassing according to claim 4.

6. A method according to one of the preceding claims, **characterized in that**, in particular, hollow fiber contactors are used for the gassing and degassing, the pore size being less than 0.1 µm, preferably less than 0.04 µm.

7. A method according to one of the preceding claims, **characterized in that** cyanobacteria are used as phototrophic microorganisms (1), preferably biofilm-forming and preferably slightly more preferably metabolizing less than 10 % of the produced organic material, in particular Gloeothece 6909, Plectonema boryanum, Anabaena sp. and Nostoc sp.

8. A method according to one of the preceding claims, wherein the methanogens (5) in the second section (4) are a mixture of acetotrophic and hydrogenotrophic archaea, wherein the acetotrophic archaea, in particular methanosarcina or synthrophobotulus, segregate the organic material from the first section (2) in carbon dioxide and hydrogen which is used by hydrogenotrophic archaea, in particular Methanocella paludicola, Methanocella arvoryzae or Methanopyrus kandleri, for biomethane production and / or mixed cultures obtained by selection from sediments of lakes and oceans, bovine pans, intestines of termites and other animals, rice fields, marshes or biogas plants.

9. A method according to one of the preceding claims, **characterized in that** inhibitors of the intracellular degradation of the organic material and / or of the intracellular carbon dioxide storage are present in the first section (2).

10. A method according to one of the preceding claims, **characterized in that** in the phototrophic microorganisms (1)
- the expression and / or the activity of the glycolic acid dehydrogenase and / or glycolic acid oxidase is suppressed;
- the carbon dioxide accumulation by carboxysomes and pyrenoids is inhibited;
- the ribulose 1,5-bisphosphate carboxylase / oxygenase and / or glycolic acid phosphate phosphatase are overexpressed;
- the ribulose 1,5-bisphosphate carboxylase / oxygenase type II is expressed;
- the excretion of organic material, especially of glycolic acid, is enhanced by the cell membrane.

11. A device for producing biogas in a combined fermenter comprising the following components:
- a first section (2), which is partially transparent, with phototrophic microorganisms (1) which are located in a medium (3);
- a second section (4) which is opaque, with methanogens (5) located in the medium (3) which is exchanged in exchange mode with the first section (2), the oxygen content being reduced;
- a connecting system (6) between the first section (2) and the second section (4);
- filters in the connecting system (6) for the gassing and degassing of the medium (3) which are connected to gas supply and gas outlets,
- a biogas effluent (7) from the second section (4);
- pumps and valves for the distribution of liquid and gas streams.

12. A device according to claim 11, **characterized in that**
- the connecting system (6) comprises a first connecting pipe (8) and a second connecting pipe (9);
- the connecting system (6) comprises a first cross-connect tube (10) interconnecting the first connecting pipe (8) and the second connecting pipe (9);
- a first filter (11) is a part of the first connecting pipe (8) between the first section (2) and the first cross-connecting pipe (10);
- a second filter (12) is a part of the second connecting pipe (9) between the first section (2) and the first cross-connecting pipe (10);
- a third filter (13) is a part of the first connecting pipe (8) between the second section (4) and the first cross-connecting pipe (10);
- a fourth filter (14) is a part of the second connecting pipe (9) between the second section (4) and the first cross-connecting pipe (10);
- a second cross-connect tube (15) connecting said first section (2) and said first cross-connect tube (10);
- a fifth filter (16) is a part of the second cross-connect tube (15) and is connected to an air line (17) with air filter (18), preferably with a pore size of 0.2 µm or less;
- an oxygen effluent (19), an oxygen supply line (20), a substitute gas supply line (21) and a substitute gas discharge line (22);
- a spare gas storage (23) and an oxygen storage (24) are present;
- a biogas filter (25) is provided as a part of the biogas effluent (7) with a subsequent biomethane storage tank (26);
- the spare gas storage (23) is connected to the biogas filter (25);
- a first pump (27) as a part of the first cross-connect tube (8) between the first section (2) and the first filter (11) and a second pump (28) as a part of the second connecting pipe (9) between the first cross-connect tube (10) and the second Filter (12) is present.

13. A device according to one of the preceding claims, **characterized in that** the first section (2) is protected from excessive solar radiation, in particular the light-transmissivie region (29) of the first section (2) can be darkened step by step.

14. A device according to one of the preceding claims, **characterized in that** the phototrophic microorganisms (1) are preferably immobilized on cellulose or a mobile carrier material, in particular gas-permeable gel capsules and the methane generators (5) preferably on activated carbon or a mobile carrier material, in particular gas-permeable gel capsules.

15. A device according to one of the preceding claims, **characterized in that** the filters are hollow fiber contactors, in particular with a gas pump (30), a permeate chamber (31), partitions (32), hollow fibers (33), an input space (34) and an output space (35).

## Revendications

1. Procédé pour la production de biogaz dans un digesteur combiné dans lequel des micro-organismes phototrophes (1) produisent dans une première section (2) de matériau organique, en particulier l'acide glycolique à partir de dioxyde de carbone et de l'oxygène et le sécrétant dans un milieu (3) (mode de production), qui est passé dans un seconde partie (4), dans laquelle des organismes à métabolisme méthanogène (5) produisent biomethane et du dioxyde de carbone dans des conditions anoxiques, **caractérisé en ce que** le milieu (3) est dégazé de l'oxygène lors du passage dé la première section (2) à la seconde partie (4) et est gazé à un retour avec de l'oxygène (mode d'échange).

2. Procédé selon la revendication 1, **caractérisé en ce que**, lorsque le contact avec la seconde section (4) est interrompu,
- le milieu (3) est dégazé par l'oxygène et est gazé par l'air de l'environnement lors d'un retour;
- l'air est ensuite utilisé par les microorganismes phototrophes (1) pour l'assimilation du carbone (mode de régénération);
- le milieu (3) est ensuite dégazé par de l'air et gazé au cours d'un retour.

3. Procédé selon la revendication 1 ou 2, **caractérisé en ce que** le rapport du dioxyde de carbone à l'oxygène dans le milieu (3) dans le mode de production situé dans la première section (2) est faible, de préférence de 1: 800 à 1: 3000.

4. Procédé selon l'une des revendications précédentes, **caractérisé en ce que** dans le mode d'échange, le milieu (3) est gazéifié avec un gaz de substitution après le dégazage d'oxygène lors du passage de la première section (2) à la seconde section), en particulier avec du dioxyde de carbone ou de l'azote, et qu'il est dégazé par le gaz de substitution avant le dégagement d'oxygène.

5. Procédé selon l'une des revendications précédentes, **caractérisé en ce que** le dioxyde de carbone est séparé du biogaz produit dans la deuxième section (4) et utilisé pour le gazage au dioxyde de carbone selon la revendication 4.

6. Procédé selon l'une des revendications précédentes, **caractérisé en ce que** l'on utilise en particulier des contacteurs à fibres creuses pour le gazage et le dégazage, la taille des pores étant inférieure à 0,1 µm, de préférence inférieure à 0,04 µm.

7. Procédé selon l'une des revendications précédentes, **caractérisé en ce que** des cyanobactéries sont utilisées comme microorganismes phototrophes (1), de préférence en biofilm et de préférence en métabolisant légèrement moins de 10% de la matière organique produite, en particulier le Gloeothece 6909, Plectonema boryanum, Anabaena sp. et Nostoc sp.

8. Procédé selon l'une des revendications précédentes, dans lequel les méthanogènes (5) de la seconde section (4) sont un mélange d'archées acétotrophes et hydrogénotrophes, où les archées acétotrophes, notamment méthanosarcine ou synthrophobotulus, séparent la matière organique de la première section (2) en dioxyde de carbone et en hydrogène utilisée par les archées hydrogénotrophes, en particulier Methanocella paludicola, Methanocella arvoryzae ou Methanopyrus kandleri, pour la production de biométhane et / ou de cultures mixtes obtenues par sélection de sédiments de lacs et d'océans, intestins de termites et autres animaux, rizières, marais ou installations de biogaz.

9. Procédé selon l'une des revendications précédentes, **caractérisé en ce que** des inhibiteurs de la dégradation intracellulaire de la matière organique et / ou du stockage intracellulaire de dioxyde de carbone sont présents dans la première section (2).

10. Procédé selon l'une des revendications précédentes, **caractérisé en ce que** dans les micro-organismes phototrophes (1)
- l'expression et / ou l'activité de l'acide glycolique déshydrogénase et / ou de l'acide glycolique oxydase est supprimée;
- l'accumulation de dioxyde de carbone par les carboxysomes et les pyrénoïdes est inhibée;
- la ribulose 1,5-bisphosphate carboxylase / oxygénase et / ou l'acide glycolique phosphate phosphatase sont surexprimées;
- la ribulose 1,5-bisphosphate carboxylase / oxygénase de type II est exprimée;
- l'excrétion de la matière organique, en particulier de l'acide glycolique, est renforcée par la membrane cellulaire.

11. Dispositif de production de biogaz dans un fermenteur combiné comprenant les composants suivants:
- une première section (2), partiellement transparente, avec des microorganismes phototrophes (1) situés dans un milieu (3);
- une deuxième section (4) opaque, avec des méthanogènes (5) situés dans le milieu (3) qui est échangé en mode échangé avec la première section (2), la teneur en oxygène étant réduite;
- un système de connexion (6) entre la première section (2) et la seconde section (4);
- des filtres dans le système de raccordement (6) pour le gazage et le dégazage du fluide (3) qui sont reliés aux sorties de gaz et de gaz,
- un effluent de biogaz (7) issu de la deuxième section (4);
- pompes et valves pour la distribution des flux de liquides et de gaz.

12. Dispositif selon la revendication 11, **caractérisé en ce que**
- le système de connexion (6) comprend un premier tuyau de raccordement (8) et un second tuyau de raccordement (9);
- le système de connexion (6) comprend un premier tube d'interconnexion (10) reliant le premier tuyau de raccordement (8) et le second tuyau de raccordement (9); '
- un premier filtre (11) fait partie du premier tuyau de liaison (8) entre le premier tronçon (2) et le premier tuyau de raccordement transversal (10);
- un deuxième filtre (12) fait partie du deuxième tuyau de liaison (9) entre le premier tronçon (2) et le premier tuyau de raccordement croisé (10);
- un troisième filtre (13) fait partie du premier tuyau de liaison (8) entre le second tronçon (4) et le premier tuyau de raccordement transversal (10);
- un quatrième filtre (14) fait partie du deuxième tuyau de liaison (9) entre le second tronçon (4) et le premier tuyau de raccordement croisé (10);
- un deuxième tube d'interconnexion (15) reliant ladite première section (2) et ledit premier tube de connexion croisée (10);
- un cinquième filtre (16) fait partie du deuxième tube de connexion croisée (15) et est relié à une conduite d'air (17) avec un filtre à air (18), de préférence avec une taille de pore de 0,2 µm ou moins;
- un effluent d'oxygène (19), une conduite d'alimentation en oxygène (20), une conduite d'alimentation en gaz de substitution (21) et une conduite de décharge de gaz de substitution (22);
- une réserve de gaz de réserve (23) et une réserve d'oxygène (24) sont présentés;
- un filtre à biogaz (25) est fourni en tant que partie de l'effluent de biogaz (7) avec un réservoir de stockage de biométhane suivant (26);
- le stockage de gaz de réserve (23) est relié au filtre à biogaz (25);
- une première pompe (27) faisant partie du premier tube d'interconnexion (8) entre la première section (2) et le premier filtre (11) et une seconde pompe (28) faisant partie du second tube de liaison 9) entre le premier tube d'interconnexion (10) et le deuxième filtre (12).

13. Dispositif selon l'une des revendications précédentes, **caractérisé en ce que** la première section (2) est protégée contre un rayonnement solaire excessif, en particulier la zone transmettant la lumière (29) de la première section (2) peut être obscurcie par pas étape.

14. Dispositif selon l'une des revendications précédentes, **caractérisé en ce que** les microorganismes phototrophes (1) sont de préférence immobilisés sur de la cellulose ou un matériau de support mobile, notamment des gélules perméables aux gaz et des générateurs de méthane (5) de préférence sur du charbon actif ou un matériau de support mobile, en particulier des gélules perméables aux gaz.

15. Dispositif selon l'une des revendications précédentes, **caractérisé en ce que** les filtres sont des contacteurs à fibres creuses, notamment une pompe à gaz (30), une chambre de perméat (31), des cloisons (32), des fibres creuses un espace d'entrée (34) et un espace de sortie (35).
